# EUROPEAN PATENT APPLICATION

(11) **EP 4 477 657 A1**
(43) Date of publication of application: **18.12.2024**
(21) Application number: 23179785.3
(22) Date of filing: 16.06.2023
(51) Int. Cl.: C07G 1/00, C07C 279/14, C07C 279/18, C08H 7/00, C08L 97/00, C07D 471/04

(54) **METHOD OF ESTERIFYING LIGNIN**

(71) Applicant: Ren Fuel K2B Ipco AB, 102 48 Stockholm (SE)
(72) Inventor: OREBOM, Alexander, Uppsala (SE); PIERROU, Clara, Uppsala (SE); MUNIER, Pierre, Uppsala (SE); DI FRANCESCO, Davide, 8820 Wädenswil (CH); SAMEC, Josef, 11350 Stockholm (SE)
(74) Representative: Brann AB

(57) **Abstract**

A method of preparing esterified lignin with fatty acids using non-toxic catalysts is presented. The present invention facilitates the production of ready to use cured lignin materials and products.

## Description

### FIELD OF INVENTION

The present invention relates to a method of esterifying lignin using a novel type of catalyst.

### BACKGROUND

There is a need for more renewable plastic materials and lignin is a potential polymer to be used.

Lignin is the most available natural polymer next to cellulose. Lignin is found in the cell walls of fibrous plants and woods along with cellulose and hemicellulose. Lignin acts as a matrix material for polysaccharides, micro-fibrils and fibres and provides strength to plant stem. It is a high molecular weight phenolic macromolecule containing three different types of monolignol monomers p-coumaryl alcohol, coniferyl alcohol and sinapyl alcohol.

WO2015/168571 discloses functionalized lignin that may be used in thermoplastics. The lignin is functionalized during reactive extraction using for example ethanol and an acid.

WO2013/050661 relates to lignin esterified with fatty acid for the production of films with improved barrier properties against oxygen and vapour. The lignin is esterified with tall oil fatty acid using acid chloride and films were made from solutions of the esterified lignin and applied to paperboard.

WO2022003073 discloses esterifying lignin with fatty acids using anhydrides and nitrogen aromatic heterocyclic catalysts such as pyridine or pyridine-based catalysts. However such catalysts are known to be toxic.

Still, there is a need to produce renewable materials in an efficient manner without using toxic or non-environmentally friendly reagents such as catalysts.

### SUMMARY OF INVENTION

The object of the present invention is to overcome the drawbacks of the prior art and provide a method of esterifying lignin using a catalyst that is efficient and less toxic or even non-toxic.

Catalysts according to the present invention are less toxic or even non-toxic which means that the obtained esterified lignin does not have to be purified to any major extent to remove the catalyst. A further advantage when using a catalyst comprising a carboxylic group such as creatine is that it will not only act as a catalyst, it will also esterify the lignin resulting in less compounds that may leak out from the material. Cured products or materials obtained from the present method facilitates that no purification steps are required and is instead ready to use.

In a first aspect the present invention relates to a method of esterifying lignin as defined in claim 1.

In a second aspect the present invention relates to a cured material obtainable by a method comprising the steps of:
a) Esterifying lignin with a fatty acid according to the method of the present invention wherein the fatty acid is a polyunsaturated fatty acid; and
b) Heating the esterified lignin at a curing temperature in the presence of crosslinking agent.

In a third aspect the present invention relates to the use of a guanidine containing compound as an esterification catalyst.

### BRIEF DESCRIPTION OF FIGURES

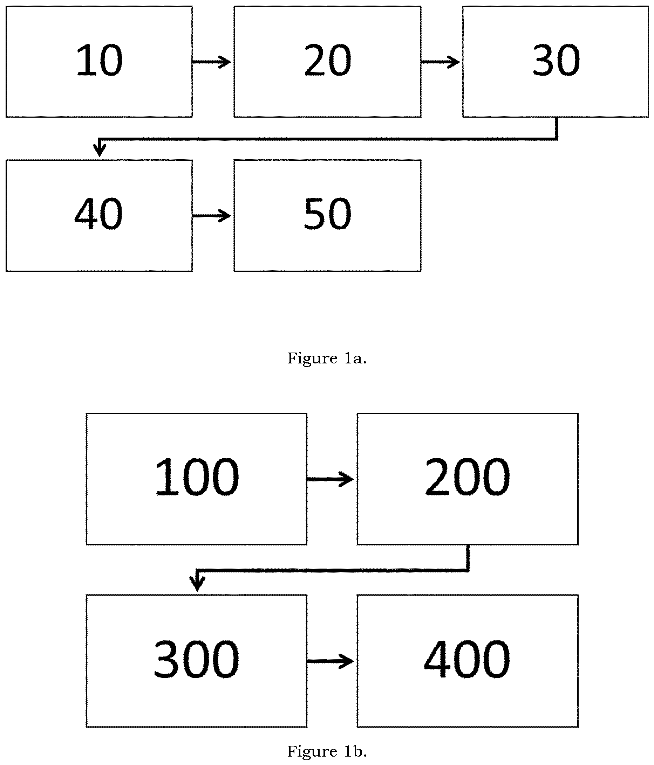

Figure 1, a) schematic illustration of the method according to the present invention, b) schematic illustration of the method according to the present invention.

### ITEMIZED EMBODIMENTS

In one embodiment of the present invention R1 to R5 is individually selected from hydrogen or aliphatic group.

In another embodiment of the present invention at least one of R1 to R4 is an aliphatic group comprising a carboxylic group.

In another embodiment of the present invention the functional group is an end group.

In another embodiment of the present invention the catalyst is selected from creatine, guanidine, arginine, dicyandiamide, diphenylguanidine 1,1,3,3-tetramethylguanidine, 1,1,2,3-tetramethylguanidine, 1,5,7-triazabicyclo[4.4.0]dec-5-ene and 7-methyl-1,5,7-triazabicyclo[4.4.0]dec-5-ene or a salt thereof.

In another embodiment of the present invention the amount of catalyst is 15pph or less based on the weight of the lignin, preferably 10pph or less, preferably 9pph or less, or 8pph or less, preferably 3pph or more, more preferably 4pph or more, more preferably 5pph or more.

In another embodiment of the present invention the fatty acid is a C12 or longer fatty acid.

In another embodiment of the present invention the fatty acid is an unsaturated fatty acid, preferably a polyunsaturated fatty acid.

In another embodiment of the present invention the weight ratio between the lignin and the fatty acid is 1:1.6 to 1:0.65, preferably 1:1.2 to 1:0.70, more preferably 1:1 to 1:0.75.

In one embodiment of the present invention an activator is added to the esterification mixture or reaction mixture, wherein the activator is preferably oxygen, zinc oxide or benzothiazyl or a combination of two or more thereof.

In another embodiment of the present invention the esterification agent is an anhydride or acyl halide, preferably acetic anhydride or acetic halide.

In another embodiment of the present invention the lignin is selected from Kraft lignin or lignin obtained from black liquor, organosolv lignin, acetosolv, steam exploded lignin or sulfonated lignin, preferably Kraft lignin or lignin obtained from black liquor, more preferably precipitated Kraft lignin or organosolv lignin.

In another embodiment of the present invention the precursor forming temperature is at least 80°C, preferably 100°C but preferably not higher than 150°C, and/or wherein the esterification temperature is at least 120°C, preferably at least 150°C, but preferably not higher than 250°C.

In one embodiment of another aspect of the present invention the crosslinking agent is selected from elemental sulphur or tetramethylthiuram disulfide (TMTD), or cumene peroxide, tert-butyl peroxide, 1,1-bis(tert-amylperoxy)cyclohexane, lauroyl peroxide, dicumene peroxide, 2,5-di(tert-butylperoxy)-2,5-dimethyl-3-hexyne, 2,5-dimethyl-2,5-di(tert-butylperoxy)hexane, di-(2-tert-butylperoxyisopropyl)benzene, butyl 4,4-di(tert-butylperoxy)valerate, 1,1-di(tert-butylperoxy)-3,3,5-trimethylcyclohexane.

In one embodiment of another aspect of the present invention the cured product is a food or drink container or a food tray.

All embodiments are applicable to all aspects and may be combined unless stated otherwise.

### DETAILED DESCRIPTION OF THE INVENTION

In the present application the term "lignin" means a polymer comprising coumaryl alcohol, coniferyl alcohol and sinapyl alcohol monomers.

In the present application the terms "weight%" and "wt%" denote the same thing and are used interchangeably.

In the present application the term "unsaturated fatty acid" encompasses monounsaturated fatty acids and polyunsaturated fatty acids.

In the present application the term "compatibilizer" denotes a compound that promotes adhesion between polymers which otherwise are less compatible. Compatibilizers are widely used to increase the miscibility of otherwise immiscible polymers or polymers that do not mix so well.

In the present invention "free fatty acid" or "free fatty acids" denotes fatty acids that are not covalently bound to lignin or triglyceride.

In the present invention "cured material" or "cured product" denotes a crosslinked polymeric material that cannot be melted. Cured material or product may be a thermoset or an elastomer.

When calculating number of repeating units and equivalents, one repeating unit of lignin is assumed to be 180 Da. The number of hydroxyl groups in the lignin is measured and calculated by preparing three stock solutions according to prior art and measured using phosphorus NMR (³¹P NMR), Varian 400 MHz. On average each monomer unit contains between 1 to 1.17 hydroxyl groups.

Molecular weight in the present application is determined using GPC (Gel Permeation Chromatography) operated at 20°C and at flow rate of 1 ml/min using THF as solvent. Polystyrene Standard RedayCal Set M(p) 250-70000 (16 standards) (Sigma product no: 76552). The columns are Styragel THF (pre-column), Styragel HR 3 THF (7.8x300 mm), Styragel HR 1 THF (7.8x300 mm), Styragel HR 0.5 THF (7.8x300 mm) all from Waters.

### Method of esterifying lignin and preparing a cured product

The method according to the present invention is a method of esterifying lignin with a fatty acid using a new catalyst. Below is the method described according to two different routes as illustrated in figure 1 and 2.

Turning to figure 1a. In step 10 are lignin, fatty acid, esterification agent and a catalyst provided. Lignin is preferably purified and has dryness of preferably at least 60wt%.

In step 20 a mixture of the fatty acid and the esterification agent is heated at a precursor forming temperature to obtain an esterification precursor. In one embodiment the esterification agent is an anhydride preferably acetic anhydride and the esterification precursor is a mixed anhydride of acetic and fatty acid i.e. an anhydride functionalized fatty acid or two fatty acids linked via an anhydride linkage.

The precursor forming temperature is preferably at least 80°C, preferably 100°C but preferably not higher than 150°C.

In step 30, the esterification precursor is mixed with the lignin to obtain an esterification mixture. The mixing may be done in any suitable way, preferably by shaking or stirring.

In step 40 is the esterification mixture heated at an esterification temperature in the presence of the catalyst, wherein the esterification temperature is preferably at least 120°C, preferably at least 150°C, preferably at least 180°C, but preferably not higher than 300°C, or preferably 250°C or lower, or preferably 220°C or lower, or preferably 200°C or lower, or more preferably190°C or lower.. The heating and the presence of the catalyst facilitates the esterification of the lignin with the fatty acid.

In the optional step 50 the obtained esterified lignin is purified. The purification is preferably done by precipitation, reducing pressure and/or by washing using a suitable solvent. In one embodiment, no purification step is performed.

Turning to figure 1b. In step 100 are lignin, fatty acid, esterification agent and a catalyst provided. Lignin is preferably purified and has dryness of preferably at least 60wt%.

In step 200, lignin, fatty acid, esterification agent and the catalyst are mixed to obtain a reaction mixture. In one embodiment the esterification agent is an anhydride preferably acetic anhydride. The mixing may be done in any suitable way preferably by shaking or stirring.

In step 300, the reaction mixture is heated at an esterification temperature to esterify lignin with fatty acid. The esterification temperature is preferably at least 120°C.

Optional step 400 comprises the step of purifying the esterified lignin. The purification is preferably done by precipitation, reducing pressure and/or by washing using a suitable solvent. In one embodiment, no purification step is performed.

Catalysts, solvents and/or any by-product may be removed in order to increase the purity of the composition. When using anhydrides such as acetic anhydride an acid is formed, e.g. acetic acid, which is preferably removed continuously during the reaction or after the reaction is completed. Catalyst and by-products may be removed using any suitable conventional technique such as evaporation preferably at reduced pressure. An advantage of the present invention is that the catalyst is less toxic or even non-toxic and therefore does not have to be removed. Further, when the catalyst comprises a carboxylic group it will not only catalyse the reaction, it will also esterify the lignin. This means that the catalyst is "consumed" at least to some extent and the need for purification is further reduced. A preferred catalyst comprising a carboxylic group is creatine.

The functionalized lignin may be isolated or purified by precipitation in for example hexane or water or by removal of solvent and catalyst through evaporation or distillation, preferably by using reduced pressure.

In one embodiment, step 200 comprises that the lignin, the free fatty acids and the catalyst are first mixed and the temperature is preferably increased to at least 90°C, preferably at least 120°C. There after the esterification agent is added and, the temperature is increased to the esterification temperature (step 300) of at least 120°C, preferably at least 150°C, preferably at least 180°C, but preferably not higher than 300°C, or preferably 250°C or lower, or preferably 220°C or lower, or preferably 200°C or lower, or more preferably 190°C or lower. The esterification temperature is maintained and pressure is then reduced to 50 mbar or lower, preferably 20 mbar or lower to distil off acetic acid. Fatty acids and esterification agent are allowed to esterify the lignin at the esterification temperature, preferably during reflux.

The catalyst used in the present method is a compound comprising a functional group having the formula of 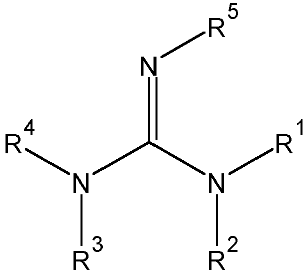 where each of R1 to R5 is individually selected from hydrogen, aliphatic or aromatic group, where R1 and R5 and/or, R2 and R3 may be linked to form a 5- or 6-member cyclic structure. When R1 and R5 and/or, R2 and R3 are linked to form a 5- or 6-member cyclic structure R1 and R5 and/or R2 and R3 are aliphatic groups, and R4 is preferably selected from a hydrogen or an aliphatic group. The catalyst preferably has a boiling point higher than 160°C, preferably higher than 180°C, more preferably higher than 200°C. Further, when using anhydride such as acetic anhydride the catalyst is believed to be acetylated which will increase the boiling temperature of the catalyst even further. A high boiling temperature allows a more efficient catalytic effect since the catalyst will remain in the reaction or esterification mixture and not evaporate.

What the present inventors found was that using the catalyst according to the present invention, lower amounts of the catalyst are needed in comparison when using pyridine-based catalysts without jeopardizing the mechanical properties.

Without being bound by theory it is believed that this is an effect of the higher boiling temperature and that the catalyst has sufficient basicity in aqueous compositions. Preferably the amount of catalyst is 15pph (parts per hundred) based on the lignin mass, preferably 10pph or less, preferably 9pph or less, or 8pph or less, preferably 3pph or more, more preferably 4pph or more, more preferably 5pph or more. In one embodiment the amount of catalyst is 10-15pph based on the lignin mass. The higher amounts are believed to facilitate tuning of the properties of the material such as viscosity and strength. In another embodiment the amount of catalyst is 3-8pph. The lower amounts are beneficial since it reduces the cost for the material and if the catalyst needs to be removed the number of purification steps may be reduced. Additionally, as seen in Example 4 and 5, a lower amount of the catalyst according to the present invention reduces the torque which means reduced viscosity and thereby better processability properties but with maintained flexural strength.

In one embodiment the functional group is an end group in the compound. In one embodiment R2 to R5 are hydrogen and R1 is an aliphatic group. Aliphatic groups may comprise heterogenous groups such as carboxylic, carbonate or ether groups. Preferred catalysts are creatine, guanidine, arginine, dicyandiamide, diphenylguanidine 1,1,3,3-tetramethylguanidine, 1,1,2,3-tetramethylguanidine, 1,5,7-triazabicyclo[4.4.0]dec-5-ene and 7-methyl-1,5,7-triazabicyclo[4.4.0]dec-5-ene or a salt thereof.

Another advantage of the present invention is that a lower amount of fatty acid is needed to obtain workable materials. The amount of fatty acids to lignin is not more than 3.0 weight equivalents, preferably 2.0 weight equivalents or less, more preferably 1.7 weight equivalents or less, more preferably 1 weight equivalent or less, but preferably 0.6 weight equivalents or higher, more preferably 0.7 weight equivalents or higher, more preferably 0.75 weight equivalents or higher, but preferably. By having a lignin to fatty acid weight ratio of 1:1.6 to 1:0.65 materials or products ranging from coatings to solid structural materials can be made. For coatings the weight ratio is preferably 1:1 to 1:1.6. Reducing the amount of fatty acid results in more solid and harder materials at a lower cost.

The fatty acid is preferably C12 or longer, more preferably C16 or longer. A nonlimiting list of fatty acids are palmitic acid, stearic acid, oleic acid, linoleic acid, linolenic acid, eicosadienoic acid and docosadienoic acid or a combination thereof.

In one embodiment the amount of fatty acid and esterification agent in the reaction mixture is such that the degree of fatty acid functionalization is at least 10%, preferably at least 20%, more preferably at least 30%, more preferably at least 40%, but preferably less than 100%, more preferably less than 80%, more preferably less than 60%, more preferably less than 50%. In one embodiment the degree of fatty acid functionalization is 20-50%. When the degree of fatty acid functionalization is lower than 100% such as less than 50% the remaining hydroxyl groups may be at least partly acetylated. In one embodiment the degree of acetylation is at least 10%, or preferably at least 20%, preferably at least 30%, more preferably at least 40% but preferably less than 90%, preferably less than 80%, more preferably less than 70%, more preferably less than 60%. The esterification agent is preferably used in excess to the number of hydroxyl groups on the lignin and the amount of esterification agent may be in excess or in deficit to the fatty acids.

A cured product or sample is prepared by reacting two unsaturated fatty acid chains esterified on the lignin. The lignin chains are crosslinked via a crosslinking compound where said crosslinking compound is a reaction product between two polyunsaturated compounds each bound to lignin chains via an ester bond. The crosslinking compound is preferably derived from two polyunsaturated fatty acids bound to each other via an ether linkage, carbon-carbon linkage, sulphur linkage or a polysulphide linkage. Said ether linkage, carbon-carbon linkage, sulphur linkage or a polysulphide linkage is formed via a reaction between the polyunsaturated fatty acids and a crosslinking agent. The sulphur linkage could be a linkage involving one or more or two or more sulphurs such as a -S- linkage or a -S-S- linkage.

Oxygen may be used as a crosslinking agent but sulphur, sulphur source, sulphur donor or peroxide is preferably used as crosslinking agent since then the curing is unexpectedly fast and provides a cured material with unexpectedly high mechanical strength. When using sulphur as the crosslinking agent oxygen is preferably also added or present during the curing. It is believed that the oxygen may act as an activator. Other suitable activators are zinc oxide and benzothiazyl. Any suitable sulphur or sulphur source or donor may be used such as elementary sulphur or tetramethylthiuram disulfide (TMTD). Any suitable peroxide may be used such as cumene peroxide, tert-butyl peroxide, 1,1-bis(tert-amylperoxy)cyclohexane, lauroyl peroxide, dicumene peroxide, 2,5-di(tert-butylperoxy)-2,5-dimethyl-3-hexyne, 2,5-dimethyl-2,5-di(tert-butylperoxy)hexane, di-(2-tert-butylperoxyisopropyl)benzene, butyl 4,4-di(tert-butylperoxy)valerate, 1,1-di(tert-butylperoxy)-3,3,5-trimethylcyclohexane. When using ethylene glycol diacrylate as the activator the present inventors have shown that it is beneficial to use a peroxide as the crosslinking agent. An advantage of using sulphur (or sulphur source or donor), or peroxide is that they may easily be mixed with the esterified lignin and thereby a better and faster curing may be accomplished. Unlike curing that is dependent on the access to oxygen these crosslinking agents are not dependent on diffusion and thereby three dimensional products may be produced.

The crosslinking density or number of crosslinking compounds per lignin chain may be varied in order to adapt the mechanical properties. Crosslinking density is given as a percentage of the number of hydroxyl groups that has been converted to ester linkages between the lignin and the crosslinking compounds and is 30-100%. Since fatty acids may be used as crosslinking compound and are commonly found as mixtures of different kind of fatty acids not all of the hydroxyl groups that are esterified are esterified with polyunsaturated fatty acids but with saturated or monounsaturated fatty acids.

What the present inventors found was that by using crosslinking compounds having more than one unsaturated group it resulted in fast curing and materials with good mechanical properties. Therefore, the crosslinking compound is polyunsaturated comprising two or more, preferably three or more unsaturated groups such as carbon-carbon double bonds. In a preferred embodiment the crosslinking compound comprises four unsaturated groups.

In order to create a sufficient distance and thereby good mechanical properties the crosslinking compound is preferably a C24 or longer compound, more preferably a C28 or longer compound, more preferably a C32 or longer compound, more preferably C36 or longer compound.

By using crosslinking compounds from renewable sources the crosslinked lignin becomes fully renewable or essentially renewable. Therefore, in a preferred embodiment the crosslinking compound is derived from natural fatty acids such as fatty acids from plants or animal fat. Fatty acids as crosslinking compounds make the material renewable and preferred fatty acids are linoleic acid, linolenic acid, eicosadienoic acid and docosadienoic acid or a combination thereof. Linoleic acid or linolenic acids are preferred since they are readily available and are found in vegetable oils.

The solid cured material according to the present invention may comprise suitable additives well-known to the person skilled in the art such as fillers, compatibilizer, lubricant, softener, anti-degradants, anti-oxidants, colour, scent and so on.

Still the solid material may be free or essentially free from any additives such as compatibilizer, lubricants, softener, anti-degradants etc. In one embodiment the amount of additives is less than 20 weight%, or less than 10 weight%, or less than 7 weight%, or less than 5 weight%, or less than 3 weight%, or less than 1 weight% but preferably 0.25 weight% or more, or 0.5 weight% or more.

Lignin according to the present invention may be any suitable lignin but is preferably selected from Kraft lignin or lignin obtained from black liquor, organosolv lignin, hydrolysis lignin, acetosolv, steam exploded lignin or sulfonated lignin, preferably Kraft lignin or lignin obtained from black liquor, more preferably precipitated Kraft lignin, more preferably acid precipitated Kraft lignin. Kraft lignin has the advantage of being commercially available in large amounts and organosolv lignin has the advantage of being easy to handle and lacks residues found in Kraft lignin.

For many industries, for example the fuel refinery industry processing lignin, the amount of metals should be as low as possible since metals may damage the machinery or disturb the process. Also halogens such as chlorides are highly toxic for the catalysts used in the refinery process and composition comprising halogens are unsuitable for such processes. Therefore the composition according to the present invention is essentially free from halogens such as chlorides. The composition according to the present invention preferably has a potassium (K) content of 100 ppm or less, preferably 50 ppm or less, and a sodium (Na) content of 100 ppm or less, preferably 50 ppm or less. In one embodiment the total metal content of the composition is 500 ppm or less, or 300ppm or less. The sulphur content may be between 2000 and 5000 ppm. The nitrogen content may be 700 ppm or less, or 500 ppm or less, or 300 ppm or less. Contents are given in relation to the amount of lignin.

The esterified lignin or the cured product obtained by the methods of the present invention may be used in many different applications. Since the obtained material comprises no or only low amounts of less toxic or non-toxic catalyst, the esterified lignin or the cured product may be used in products that are in contact with food such as food or drink container or food tray.

### EXAMPLES

### Example 1

General procedure of preparing kraft lignin ester material according to the present invention: To a 1 L round bottomed flask equipped with a mechanical stirrer was added fatty acid (90 g), mixture was heated to 60 °C and dry kraft lignin (100.0 g) was added. Temperature was increased to 90 °C and a catalyst (76.2 mmol) was added. Temperature was increased to 130 °C and acetic anhydride (56 mL) was added over 5 minutes and temperature of oil bath was raised to 190 °C. The reaction was refluxed for 1 h and then the pressure was lowered over 1.5 hours to 30 mbar to distil off acetic acid. Reaction was further continued for 60 min at 30 mbar to afford a homogenous liquid.

### Example 2

In order to assess the quality of esterified lignin, two extraction protocols are conducted. In the first protocol, THF is used as the extraction medium and will dissolve the esterified lignin material as well as unreacted or partially reacted lignin and fatty acid. The precipitate, or THF-insolubles, will contain polymerized lignin with high molecular weight and impurities, and its mass is used as a quality indicator for the esterified lignin. In the second protocol, pentane is used as the extraction medium and will dissolve the unreacted free fatty acid. The precipitate will contain the esterified lignin and its mass is used as an estimate of the transesterification yield.

Materials were prepared according to Example 1 using linoleic acid as fatty acid, acetic acid anhydride as esterification agent and four different catalysts. Two reference samples (Ref. 1 and 2) were also prepared using the linoleic acid, acetic anhydride and 4-methylpyridine as catalyst.

Extraction of the material using THF: Two samples of around 5 g of each material were shaken with THF for at least one hour in 50 mL plastic centrifugation tubes. The obtained slurries were centrifugated for 5 minutes and the supernatants were discarded. The precipitates were washed 3 additional times with THF using the same procedure. The final precipitates, or THF-insolubles, were dried overnight at 60 °C and weighed against the mass of the initial sample.

Extraction of the material using pentane: Four samples of around 1 g of each material were shaken with 5 mL THF for at least one hour in 50 mL plastic centrifugation tubes. Pentane was added and the tubes were shaken for a few seconds. The obtained slurries were centrifugated for 5 minutes and the supernatants were discarded. The precipitates were washed 2 additional times with pentane using the same procedure. The final precipitates were dried overnight at 60 °C and weighed against the mass of the initial sample. From these values, the amount of free fatty acid solubilized by pentane, and therefore the kraft lignin transesterification yield, were calculated.

Reference samples (Ref 1 and 2) in which the catalyst was replaced with 4-methylpyridine underwent the same procedure. The results are summarized in Table 1.

**Table 1. Amount of catalyst is given as parts per hundred (pph) relative to lignin mass.**

| **Material** | **Catalyst** | **Catalyst Amount (pph)¹** | **THF-insolubles (%)** | **Yield (%)²** |
|---|---|---|---|---|
| 1 | Guanidinium carbonate | 13.8 | 3.04 ± 0.01 | 34 ± 2 |
| 2 | Dicyandiamide | 6.4 | 1.18 ± 0.02 | 44 ± 2 |
| 3 | Creatine | 10 | 3.29 ± 0.01 | 32 ± 6 |
| 4 | Arginine | 13.2 | 5.74 ± 0.18 | 28 ± 3 |
| Ref 1 | 4-methylpyridine | 10 | 4.40 ± 0.07 | 36 ± 2 |
| Ref 2 | 4-methylpyridine | 33 | 2.60 ± 0.01 | 42 ± 1 |

| | | | | |
|---|---|---|---|---|
| ¹Amount relative to kraft lignin ²Transesterification yield | | | | |

### Example 3

Screening of lignin to fatty acid weight ratios: The procedure described in Example 1 was performed with varying kraft lignin to fatty acid ratios (oleic acid, technical grade), as well as fixed stoichiometric amounts of creatine (catalyst) and acetic anhydride, relative to the added kraft lignin amount. Amount of THF-insoluble components was obtained according to the method disclosed in Example 2. Table 2 summarizes the prepared materials.

**Table 2, FA is oleic acid. Creatine is added in an amount of 10pph relative to the lignin mass.**

| **Material** | **Lignin:FA ratio** | **Kraft lignin (g)** | **Fatty acid (g)** | **Creatine (g)** | **Acetic anhydride (mL)** | **THF-insolubles (%)** |
|---|---|---|---|---|---|---|
| 5 | 1:3 | 50 | 150 | 5 | 37 | 0.67 |
| 6 | 1:2 | 65 | 130 | 6.5 | 48 | 0.75 |
| 7 | 1:1.6 | 75 | 120 | 7.5 | 56 | 0.83 |
| 8 | 1:1.2 | 90 | 108 | 9 | 67 | 1.38 |
| 9 | 1:0.9 | 100 | 90 | 10 | 74 | 1.96 |
| 10 | 1:0.75 | 110 | 83 | 11 | 81 | 1.60 |
| 11 | 1:0.6 | 120 | 72 | 12 | 89 | N/A (gel) |

| | | | | | | |
|---|---|---|---|---|---|---|
| Lignin = Kraft lignin FA=Fatty acid, oleic acid | | | | | | |

### Example 4

The aim was to prepare resin formulations containing kraft lignin ester of linoleic acid (Esterified lignin) with sulfur, using zinc oxide and benzothiazyl disulfide as activators. The resins were cured/vulcanised and evaluated with regards to flexural strength and other physical properties.

Preparation and curing of resin: Esterified lignin was prepared as described in Example 1 and was milled using a Retsch GM300 mixer at 4000 rpm for 10 seconds. The milled esterified lignin (60 g), elementary sulphur (2.4 g), zinc oxide (3 g) and benzothiazyl disulfide (1.2 g) were added to an internal mixer (Measuring mixer 50 EHT, Brabender, Germany) equipped with Banbury blades and pre-heated at 120 °C. The contents were mixed for 5 minutes at 100 rpm and the mean torque value after 4 minutes was recorded to assess the viscosity and processability of the mixture (see Table 3), where lower torque values are desirable as they are synonym of lower viscosity and easier processability. After cooling somewhat, the obtained paste was divided into six parts that were loaded into a rectangular Teflon mould (specimen size, 43x18x3 mm). The mould was covered with 1 mm silicone sheet, compressed together between aluminium plates to obtain an airtight seal, and cured at 150 °C for 30 minutes.

### Example 5

Cured rectangular specimens were prepared according to Example 4. Flexural properties were evaluated using a SAUTER TVS 5000N240 motorised test stand (KERN & SOHN GmbH, Germany), equipped with a custom three-point bending fixture. The average flexural strengths were calculated from the obtained forces at break and are summarized in Table 3:

**Table 3. Amount of catalyst is given as parts per hundred (pph) relative to lignin mass and as molar quantity per gram of lignin (mmol/g).**

| **Catalyst** | **Catalyst amount** | | **Mean torque³ (N.m)** | **Flexural strength (MPa)** |
|---|---|---|---|---|
| | **pph¹** | **mmol/g²** | | |
| Guanidinium carbonate | 13.8 | 76.2 | 14.4 | 7 ± 1 |
| Dicyandiamide | 6.4 | 76.2 | 10.8 | 7 ± 1 |
| Creatine | 5 | 38.1 | 7.4 | 8 ± 3 |
| Creatine | 10 | 76.2 | 13.5 | 7 ± 2 |
| Arginine | 13.2 | 76.2 | 13.2 | 7 ± 2 |
| 4-methylpyridine | 33 | 354.3 | 9.5 | 10 ± 4 |

| | | | | |
|---|---|---|---|---|
| ¹Amount relative to kraft lignin ²Molar quantity per gram of kraft lignin ³Mean torque after 4 min. mixing at 120 °C | | | | |

## Claims

1. A method of esterifying lignin with fatty acid comprising:
a. Providing lignin, fatty acid, esterification agent and a catalyst;
b. Esterifying the lignin with the fatty acid by either
i. Heating a mixture of the fatty acid and the esterification agent at a precursor forming temperature to obtain an esterification precursor;
ii. Mixing the esterification precursor with the lignin to obtain an esterification mixture; and
iii. Heating the esterification mixture at an esterification temperature in the presence of the catalyst to obtain esterified lignin;
or by
I. Mixing the lignin, the fatty acid, the esterification agent and the catalyst to obtain a reaction mixture; and
II. Heating the reaction mixture at an esterification temperature to obtain esterified lignin;
wherein the catalyst is a compound comprising a functional group having the formula of 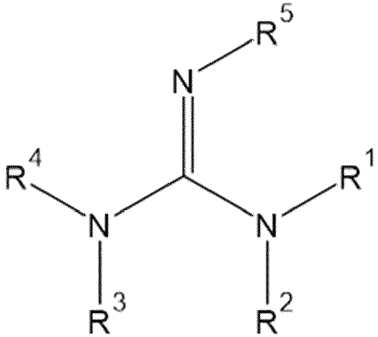 wherein each R1 to R5 is individually selected from hydrogen, aliphatic or aromatic group, wherein R1 and R5 and/or, R2 and R3 may be linked to form a 5- or 6-member cyclic structure, and wherein R4 preferably is hydrogen or an aliphatic group.

2. The method according to claim 1 wherein R1 to R5 is individually selected from hydrogen or aliphatic group.

3. The method according to claim 1 or 2 wherein at least one of R1 to R4 is an aliphatic group comprising a carboxylic group.

4. The method according to any one of claim 1 to 3 wherein the functional group is an end group.

5. The method according to any one of claim 1 to 4 wherein the catalyst is selected from creatine, guanidine, arginine, dicyandiamide, diphenylguanidine 1,1,3,3-tetramethylguanidine, 1,1,2,3-tetramethylguanidine, 1,5,7-triazabicyclo[4.4.0]dec-5-ene and 7-methyl-1,5,7-triazabicyclo[4.4.0]dec-5-ene or a salt thereof.

6. The method according to any one of the preceding claims wherein the amount of catalyst is 15pph or less based on the weight of the lignin, preferably 10pph or less, preferably 9pph or less, or 8pph or less, preferably 3pph or more, more preferably 4pph or more, more preferably 5pph or more.

7. The method according to any one of the preceding claims wherein the weight ratio between the lignin and the fatty acid is 1:1.6 to 1:0.65, preferably 1:1.2 to 1:0.70, more preferably 1:1 to 1:0.75; and wherein the fatty acid preferably is a C12 or longer fatty acid, preferably a polyunsaturated fatty acid.

8. The method according to any one of the preceding claims wherein the esterification agent is an anhydride or acyl halide, preferably acetic anhydride or acetic halide.

9. The method according to any one of the preceding claims wherein the lignin is selected from Kraft lignin or lignin obtained from black liquor, organosolv lignin, acetosolv, steam exploded lignin or sulfonated lignin, preferably Kraft lignin or lignin obtained from black liquor, more preferably precipitated Kraft lignin or organosolv lignin.

10. The method according to any one of the preceding claims wherein the precursor forming temperature is at least 80°C, preferably 100°C but preferably not higher than 150°C, and/or wherein the esterification temperature is at least 120°C, preferably at least 150°C, but preferably not higher than 250°C.

11. A method of preparing a cured material comprising:
a. Esterifying lignin with fatty acid according to any one of claim 1 to 10 wherein the fatty acid is a polyunsaturated fatty acid; and
b. Heating the esterified lignin at a curing temperature in the presence of crosslinking agent.

12. The method according to claim 11 wherein the crosslinking agent is selected from elemental sulphur or tetramethylthiuram disulfide (TMTD), or cumene peroxide, tert-butyl peroxide, 1,1-bis(tert-amylperoxy)cyclohexane, lauroyl peroxide, dicumene peroxide, 2,5-di(tert-butylperoxy)-2,5-dimethyl-3-hexyne, 2,5-dimethyl-2,5-di(tert-butylperoxy)hexane, di-(2-tert-butylperoxyisopropyl)benzene, butyl 4,4-di(tert-butylperoxy)valerate, 1,1-di(tert-butylperoxy) -3,3,5-trimethylcyclohexane.

13. A cured product obtainable by the method according to any one of claim 11 to 12.

14. The cured product according to claim 13 wherein said product is a food or drink container or a food tray.

15. Use of a guanidine containing compound as an esterification catalyst.
